Europäisches Patentamt

European Patent Office

Office européen des brevets

(19)

(11) Veröffentlichungsnummer: **0 257 444 B1**

# EUROPÄISCHE PATENTSCHRIFT

(45) Veröffentlichungstag der Patentschrift: **18.09.91**

(21) Anmeldenummer: **87111649.7**

(22) Anmeldetag: **12.08.87**

(51) Int. Cl.⁵: **C08F 226/10**, C08F 220/12, A61K 7/11

(54) **Terpolymerisate, ihre Verwendung in Haarbehandlungsmitteln und diese enthaltende Haarbehandlungsmittel.**

(30) Priorität: 18.08.86 DE 3627970

(43) Veröffentlichungstag der Anmeldung:
02.03.88 Patentblatt 88/09

(45) Bekanntmachung des Hinweises auf die
Patenterteilung:
18.09.91 Patentblatt 91/38

(84) Benannte Vertragsstaaten:
DE ES FR GB IT NL

(56) Entgegenhaltungen:
EP-A- 0 100 890
US-A- 3 405 084

(73) Patentinhaber: **BASF Aktiengesellschaft**
**Carl-Bosch-Strasse 38**
**W-6700 Ludwigshafen(DE)**

(72) Erfinder: **Nuber, Adolf, Dr.**
**Kurt-Schumacher-Strasse 11**
**W-6737 Boehl-Iggelheim(DE)**
Erfinder: **Sanner, Axel, Dr.**
**Lorscher Ring 2 c**
**W-6710 Frankenthal(DE)**
Erfinder: **Straub, Ferdinand, Dr.**
**Ziegelstrasse 20**
**W-6832 Hockenheim(DE)**
Erfinder: **Vogel, Friedrich, Dr.**
**Im kleinen Letten 3**
**W-6706 Wachenheim(DE)**

EP 0 257 444 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Terpolymeres aus Vinylpyrrolidon, tert. -Butyl(meth)acrylat und Acryl- oder Methacrylsäure, dessen Verwendung in Haarbehandlungsmitteln und diese enthaltende Zusammensetzungen für die Haarkosmetik.

Filmbildende Harze auf der Basis von carboxylgruppenhaltigen Vinylmonomeren und (Meth)Acrylester und/oder Vinylacetat werden seit langem insbesondere für Haarsprays, bei denen Halogenkohlenwasserstoffe, wie Fluorkohlenwasserstoffe, als Treibmittel verwendet werden, eingesetzt. Für die praktische Anwendung ist dabei zweckmäßig, mindestens einen Teil der Carboxylgruppen zu neutralisieren, beispielsweise mit einem Amin oder einer Aminohydroxyverbindung, wie in den US-PS 2 996 471, 3 405 084 oder 3 577 517 beschrieben wird. Mit der wenigstens teilweisen Neutralisation werden die Wasserlöslichkeit oder Dispergierbarkeit des verwendeten Harzes verbessert und eine leichtere Entfernbarkeit aus dem Haar beim Waschen wird ermöglicht. Die Neutralisation gestattet es, die Flexibilität der aufgesprühten Filme günstig zu beeinflussen.

Ökologische Gründe führen in zunehmendem Maße zu einem Austausch der Halogenkohlenwasserstoffe in Aerosol-Haarsprayzubereitungen durch reine Kohlenwasserstoffe, wie Propan oder Butan. Die Verwendung dieser Kohlenwasserstoffe als Treibmittel bringt eine Reihe von Problemen mit sich, wobei die herabgesetzte Löslichkeit der Haarsprayharze, beispielsweise in alkoholischer Lösung in Gegenwart von diesen Kohenwasserstoffen als Treibmittel eine besondere Rolle spielt. Obwohl die carboxylierten Harze in wasserfreien Alkohol/Halogenkohlenwasserstoff-Sprayformulierungen in der Regel gut löslich sind, kann ihre verminderte Löslichkeit in den neuerdings verwendeten Alkohol/Kohlenwasserstoff-Formulierungen dazu führen, daß sie für moderne Haarsprayformulierungen nicht mehr akzeptabel sind.

In der US-PS 3 405 084 werden neutralisierte Terpolymerisate für Haarsprayformulierungen beschrieben, die aus 20 bis 75 Gew.-% Vinylpyrrolidon, 20 bis 70 Gew.-% eines Alkylesters der Acryl-oder Methacrylsäure mit einem gesättigten geradkettigen oder verzweigten Rest von 1 bis 10 C-Atomen im Esteralkohol und 3 bis 25 Gew.-% Acrylsäure oder Methacrylsäure bestehen.

Als einpolymerisierte Acrylate werden beispielsweise Propyl-, Isopropyl-, Butyl-, Isobutyl-acrylate und -methacrylate in der Beschreibung genannt und in den Ausführungsbeispielen Terpolymere mit Ethylmethacrylat, Ethylacrylat, Butylmethacrylat, Hexylmethacrylat und Butylacrylat beschrieben.

Nachteilig bei der praktischen Anwendung, vor allem in Haarsprays an diesen in der US-PS 3 405 084 tatsächlich beschrieben Terpolymeren ist, daß sie teilweise eine unbefriedigende, nicht ausreichende Löslichkeit in den heute aus ökologischen Gründen bevorzugten Treibgasen, wie Propan, Butan, Isopentan oder ihren Mischungen davon, aufweisen, obwohl auch aus diesem Stand der Technik in allgemeiner Form, Spalte 7, Zeile 49, Kohlenwasserstoffe als Treibmittel hervorgehen. Andere dieser beschriebenen Terpolymeren, die eine genügende hohe Löslichkeit aufweisen, haben den Nachteil, daß sie zur Klebrigkeit, die einer Anwendung in der Kosmetik entgegensteht, neigen und eine nicht ausreichende Curl Retention zeigen.

Die EP-A-100 890 betrifft Copolymerisate aus (a) einem $C_2$- bis $C_{20}$-Alkylester der (Meth)acrylsäure, z.B. tert.-Butyl(meth)acrylat, (b) einem stickstoffhaltigen, neutral reagierenden, wasserlöslichen Monomeren, z.B. N-Vinylpyrrolidon, (c) einem kationische Gruppen enthaltenden Monomeren, z.B. N-Vinylimidazol, und (d) einer olefinisch ungesättigten $c_3$- bis $C_5$-Carbonsäure, z.B. (Meth)acrylsäure. Diese Copolymerisate werden als Filmbildner in Haarbehandlungsmitteln empfohlen. Die Copolymerisate weisen eine gute Verträglichkeit mit polaren Lösungsmitteln und unpolaren Treibgasen auf.

Es bestand daher die Aufgabe, Terpolymere aufzuzeigen, bei denen die obengenannten Schwierigkeiten, betreffend die Löslichkeit in Kohlenwasserstoff-Treibmitteln bzw. in Aerosol-Formulierungen, eine bessere Curl Retention und die Bildung klebefreier Filme auf dem Haar, überwunden werden können.

Die Lösung der Aufgabe besteht in einem Terpolymer, erhalten durch radikalische Polymerisation von

| | |
|---|---|
| 20 bis 50 Gew.-% | Vinylpyrrolidon, |
| 40 bis 70 Gew.-% | tert.-Butylacrylat oder tert.-Butyl-methacrylat und |
| 2 bis 15 Gew.-% | Acrylsäure oder Methacrylsäure |

und dessen Carboxylgruppen gegebenfalls zu 5 bis 100 % , bevorzugt 50 bis 90 %, durch ein organisches Amin neutralisiert sind und das einen K-Wert von 10 bis 60 aufweist.

Als bevorzugte Bereiche für das erfindungsgemäße Terpolymer sind zu nennen

| | |
|---|---|
| 25 bis 40 Gew.-% | Vinylpyrolidon, |
| 40 bis 70 Gew.-% | tert.-Butylacrylat oder tert.-Butylmethacrylat und |
| 5 bis 10 Gew.-% | Acrylsäure oder Methacrylsäure. |

Bei Verwendung der Comonomeren tert.-Butylacrylat oder -methacrylat wird ein Terpolymerisat erhalten, das sowohl im Hinblick auf die Löslichkeit, insbesondere in den in Haarsprays verwendeten Alkohol-Kohlenwasserstoff-Treibmittelgemischen, als auch im Hinblick auf die filmbildenden Eigenschaften in nicht vorhersehbarer Weise ein Optimum darstellt und die gestellten Anforderungen erfüllt. Das gilt vor allem, nachdem sich die Copolymeren mit n-Butyl- und Isobutyl-Acrylat und -methacrylat im praktischen Versuch auf Treibgasverträglichkeit und Curl Retention als teilweise unbefriedigend erwiesen haben.

Die erfindungsgemäßen Terpolymerisate eigen sich als Filmbildner insbesondere für Haarsprays, aber auch in Haarfestigern, -schäumen oder -gelen können sie verwendet werden.

Die erfindungsgemäßen Terpolymere liegen zweckmäßigerweise in durch ein organisches Amin teilweise oder vollständig neutralisierten Form vor, wobei die Carboxylgruppen zu 5 bis 100 % neutralisiert sind. Bevorzugt ist eine Teilneutralisation der Carboxylgruppen von 50 bis 90 %. Die Neutralisation der Carboxylgruppen erfolgt bevorzugt mit einem Alkanolamin aus der Reihe der Mono-, Di- oder Trialkanolamine mit 2 bis 5 C-Atomen im Alkanolrest, wie Mono-, Di- oder Triethanolamin, Mono-, Di-oder Tripropanolamin oder 2-Amino-2-methyl-propanol, oder Alkandiolaminen mit 2 bis 4 C-Atomen im Alkandiolrest, wie 2-Amino-2-methyl-1,3-propandiol oder 2-Amino-2-ethyl-1,3-propandiol, oder durch Di(methoxyethyl)amin oder durch primäre, sekundäre und tertiäre Alkylamine mit insgesamt 5 bis 10 C-Atomen, wie N,N-Diethylpropylamin.

Davon sind 2-Amino-2-methyl-propanol, Triisopropanolamin und 2-Amino-2-ethyl-1,3-propandiol bevorzugt.

Gegenstand der Erfindung ist auch ein Haarbehandlungsmittel, das als Filmbildner ein erfindungsgemäßes Terpolymer in der teil- oder vollständig neutralisierten Form in einer Menge von 0,5 bis 15, bevorzugt 1 bis 10 %, bezogen auf das Gesamtgewicht, enthält, und die Verwendung der erfindungsgemäßen Terpolymeren in Haarbehandlungsmitteln.

Die besonders bevorzugte Zubereitung stellt eine Haarsprayzusammensetzung dar, dadurch gekennzeichnet, daß sie

| | |
|---|---|
| 0,5 bis 15 Gew.-% | erfindungsgemäßes Terpolymer, dessen Carboxylgruppen zu 5 bis 100 %, bevorzugt 50 bis 90 %, durch ein obengenanntes organisches Amin neutralisiert sind, |
| 10 bis 95 Gew.-% | eines Lösungsmittels, ausgewählt aus der Gruppe Aceton, Ethanol, Propanol, Isopropanol und 1-Methoxypropanol-2 oder deren Gemische und |
| 5 bis 90 Gew.-% | eines Treibmittels, ausgewählt aus der Gruppe Propan, n-Butan, iso-Butan, 2,2-Dimethylpropan, iso-Pentan und Dimethylether und deren Gemische |

enhält.

Gegebenenfalls enthält diese Haarsprayzusammensetzung, bezogen auf das Gesamtgewicht, zusätzlich 0,1 bis 5 Gew.-% Wasser oder 1 bis 35 Gew.-% Methylenchlorid oder Trichlorethan.

Die bevorzugten Zusammensetzungen weisen

| | |
|---|---|
| 1 bis 10 Gew.-% | erfindungsgemäßes Harz, |
| 15 bis 60 Gew.-% | Lösungsmittel und |
| 40 bis 85 Gew.-% | Treibgas auf. |

Die erfindungsgemäßen Terpolymere der oben angegebenen und in den Beispielen hervorgehobenen Zusammensetzungen können vorteilhaft und bevorzugt in Haarsprays der obigen Rahmenvorschrift verwendet werden.

Wenn auf eine besonders gute Haarfestigermischung Wert gelegt wird, empfehlen sich solche Terpolymere, die Methacrylsäure enthalten und die gleichzeitig die höchsten Werte an Treibgasverträglichkeit aufweisen.

Daneben kann die Wasserlöslichkeit der verwendeten Terpolymere im Hinblick auf Zubereitungen, die Wasser als Lösungsmittel enthalten, eine wichtige Rolle spielen. Solche Zubereitungen sind insbesondere Haarfestiger, Haarschäume und Haargele. Für derartige Zwecke empfehlen sich innerhalb der angegebenen Bereiche höhere Vinylpyrrolidon- und Acrylsäure- oder Methacrylsäuregehalte, wie etwa den Beispielen 1 bis 9 entsprechend.

Eine zweckmäßige Zubereitung für Haarfestiger enthält

| | |
|---|---|
| 1 bis 15 Gew.-% | erfindungsgemäße Terpolymer, dessen Carboxylgruppen zu 5 bis 100 %, bevorzugt 50 bis 90 %, durch ein obengenanntes organisches Amin neutralisiert sind, |
| 0 bis 99 Gew.-% | eines Lösungsmittels, ausgewählt aus der Gruppe Aceton, Ethanol (96 %ig), Propanol, Isopropanol und 1-Methoxypropanol-2 oder deren Gemische und |
| 0 bis 99 Gew.-% | Wasser. |

Eine bevorzugte Haarfestiger-Zusammensetzung ist überwiegend wäßrig und enthält 2 bis 10 Gew.-% oben definiertes Terpolymer und 60 bis 98 Gew.-% Wasser und gegebenenfalls als Rest zu 100 Gew.-% eines der obengenannten Lösungsmittel oder deren Gemisch. Die Herstellung von wäßrigen Zubereitungen stellen einen besonderen Vorteil der vorliegenden Erfindung dar.

Eine zweckmäßige und vorteilhafte Zusammensetzung für Haarschäume ergibt sich nach folgender Vorschrift:

1 bis 15 Gew.-%,    bevorzugt 5 bis 10 Gew.-%, erfindungsgemäßes Terpolymer, dessen Carboxylgruppen zu 5 bis 100 %, bevorzugt 50 bis 90 Gew.-%, durch ein obengenanntes Amin neutralisiert sind,

5 bis 90 Gew.-%,    bevorzugt 60 bis 80 Gew.-% Wasser

0 bis 15 Gew.-%,    bevorzugt 5 bis 10 Gew.-% eines Lösungsmittels aus der Gruppe Aceton, Ethanol, Propanol, Isopropanol und 1-Methoxypropanol-2-oder deren Gemische, und

50 bis 20 Gew.-%    eines Treibmittels, ausgewählt aus der Gruppe bestehend aus Propan, n-Butan, iso-Butan, 2,2-Dimethylpropan, iso-Pentan und Dimethylether und deren Gemische, wovon Mischungen aus Propan/Butan (25:75) bevorzugt sind.

Diesen Zusammensetzungen werden, bezogen auf das Gesamtgewicht, 0,5 bis 1 Gew.-% dem Fachmann an sich bekannte Hilfsmittel zur Schaumbildung und Schaumstabilisierung zugesetzt. Beispielsweise seien genannt Ceteareth 25 oder 11, Nonoxynol 14 oder 10 oder Cocamide DEA (vgl. CTFA, Cosmetics Ingredient Dictionary, 3. Ausgabe 1982).

Selbstverständlich kommen für die obengenannten verschiedenen Zubereitungsformen auch übliche Zusätze, wie Parfüm, Konservierungsstoffe u.a., in Betracht.

Daraus ergibt sich, daß Haarbehandlungsmittel auf wäßriger Basis ein bevorzugter Gegenstand der vorliegenden Erfindung sind. Für Haar-Zubereitungen auf wäßriger Basis eignen sich insbesondere die Terpolymeren, die aus Vinylpyrrolidon, tert.-Butylacrylat und Methacrylsäure zusammengesetzt sind, in einer Menge von 2 bis 10 Gew.-%, bezogen auf das Gesamtgewicht.

Die erfindungsgemäßen Copolymeren werden durch übliche radikalische Copolymerisation bevorzugt in Form einer Lösungspolymerisation in einem einwertigen niederen Alkohol mit 1 bis 4 Kohlenstoffatomen, insbesondere Ethanol oder Isopropanol, hergestellt. Als Initiatoren werden die üblichen Peroxide, wie Benzoylperoxid, tert.-Butylperpivalat, tert.-Butylper-2-ethylhexanoat, Di-tert.-Butylperoxid, tert.-Butylhydroperoxid, sowie Azostarter, wie Azo-bis-Isobutyronitril, zweckmäßig in Mengen 0,3 bis 2,0 Gew.-%, bezogen auf das Gewicht der Monomeren, verwendet.

Nach der Polymerisation liegen in der Regel 30 bis 80 gew.-%ige Lösungen vor. Falls erforderlich, kann das Lösungsmittel auf übliche Weise, z.B. durch Sprühtrocknung, entfernt werden.

Die erfindungsgemäßen Terpolymeren weisen K-Werte von 10 bis 60, bevorzugt 15 bis 50, gemessen in 2 gew.-%iger Lösung in Ethanol bei 25°C in der Säureform, auf. Der K-Wert ist ein Maß für das Molgewicht (Fikentscher, Cellulosechemie 13, (1932) Seite 58 bis 64 und 71 bis 74).

Die nun folgenden Beispiele erläutern die Erfindung. Die in den Beispielen genannten Teile und Prozente beziehen sich auf das Gewicht.

Herstellvorschrift für Beispiele 1 und 2 und Vergleichsbeispiele 3 bis 8:

100 Teile Monomerengemisch der Zusammensetzung gemäß den Beispielen der Tabelle 1 werden in 77 Teilen Isopropanol gelöst. In einem Rührkolben mit Rückflußkühler und 2 Tropftrichtern werden 10 Gew.-% dieser Monomerenlösung, 10 Gew.-% der vorbereiteten Starterlösung aus 0,8 Teilen tert.-Butylperpivalat und 12 Teilen Isopropanol vorgelegt und auf ca. 75°C aufgeheizt.

Nach dem Anpolymerisieren, erkennbar an einer Viskositätserhöhung, wird die restliche Monomerlösung innerhalb von ca. 5 h und gleichzeitig die restliche Starterlösung innerhalb von etwa 7 Stunden zugegeben, wobei die Innentemperatur bei schwachem Sieden auf ca. 80 bis 82°C gehalten wird. Nach dem Ende der Zugabe wird noch ca. 1 Stunde bei diesen Temperaturen nachpolymerisiert.

Beispiele 9 bis 12

100 Teile Monomerengemisch gemäß der Zusammensetzung nach den Beispielen 9 bis 12 in Tabelle 2 werden in 24 Teilen Ethanol gelöst. In einem Rührkolben mit Rückflußkühler und 2 Tropftrichtern werden 10 Gew.-% dieser Monomerenlösung, 10 Gew.-% der vorbereiteten Starterlösung aus 0,8 Teilen tert.-Butylperpivalat und 27 Teilen Ethanol und 50 Teile Ethanol vorgelegt und auf ca. 75°C aufgeheizt.

Nach dem Anpolymerisieren, erkennbar an einer Viskositätserhöhung, wird die restliche Momonerlösung innerhalb von 3 Stunden und gleichzeitig die restliche Starterlösung innerhalb von etwa 6 Stunden zugegeben, wobei die Innentemperatur auf ca. 78 bis 80°C gehalten wird.

Nach dem Ende der Zugabe wird noch ca. 1 Stunde bei diesen Temperaturen nachpolymerisiert.

Vergleichsbeispiele 3 bis 8:

Zum Vergleich wurden unter den Bedingungen des Beispiels 1 Terpolymere mit n-, sec.- und iso-Butylacrylat sowie n-, sec.-und iso-Butyl-methacrylat hergestellt, wie aus der Tabelle ersichtlich und sie sich aus der US-PS 3 405 084 ergeben.

An den verschiedenen Terpolymerisaten, deren Carboxylgruppen zu 75 % mit 2-Amino-2-methyl-propanol neutralisiert wurden, wurden die Treibgasverträglichkeit und die Curl Retention bestimmt. Die Ergebnisse sind in den Tabellen 1 und 2 zusammengefaßt.

Die Treibgasverträglichkeit mit Propan/Butan im Gewichtsverhältnis 40:60 gibt an, wieviel Gew.-% dieses Treibgasgemisches in einer ethanolischen Haarspray-Lösung mit 2 Gew.-% Terpolymerisat, neutralisiert zu 75 %, verwendet werden kann, um bei 0°C noch eine Klare Lösung zu erhalten. Eine Treibgasverträglichkeit von z.B. 71 % bedeutet, daß eine 3 gew.-%ige Lösung mit 71 Gew.-% Treibgas und Rest zu 100 Gew.-% Ethanol bei 0°C eine klare Lösung ergibt.

Die Curl Retention ist ein Maß für die Haarfestigungswirkung. Sie wird im Modellversuch gemessen an Haarlocken, erzeugt durch eine übliche Wasserwelle an ca. 15 cm langen Haaren, die mit 2 gew.-%iger Spraylösung eines Harzes gemäß Tabelle 1 oder 2 und zu 75 % teilneutralisiert aus 10 cm Entfernung 4 sec. lang besprüht werden. Nach 5-stündiger Behandlung der aufgehängten Locken in einer Klimakammer (25°C, 90 % relative Luftfeuchte) wird die relative Verformung (Aufweitung) der Locken, bezogen auf die ursprüngliche Form, festgestellt. Ein hoher Wert bedeutet hohe Festigungswirkung, d.h. 100 % wäre Erhalt der ursprünglichen Form der aufgehängten Locke, 0 % wäre ein völlig gestrecktes Haar.

Tabelle 1

| Bsp. Nr. | Zusammensetzung (Gew.-%) | Treibgasverträglichkeit (Propan/Butan 40:60, in Ethanol abs, 0°C) | Curl Retention (25°C, 90 % rel. Feuchte, 5 h) | K-Wert (2 gew.-%ig in Ethanol, 25°C) |
|---|---|---|---|---|
| 1. | 40 VP/50 tert.-BA/10 AS | 71 % | 56 % | 20 |
| 2. | 40 VP/50 tert.-BMA/10 AS | 64 % | 75 % | 22 |
| **Vergleichsbeispiel-Nr.** | | | | |
| 3. | 40 VP/50 n-BA/10 AS | 65 % | 29 % | 20 |
| 4. | 40 VP/50 sec.-BA/10 AS | 68 % | 24 % | 19 |
| 5. | 40 VP/50 iso-BA/10 AS | 68 % | 27 % | 19 |
| 6. | 40 VP/50 n-BMA/10 AS | 58 % | 57 % | 22 |
| 7. | 40 VP/50 sec.-BMA/10 AS | 61 % | 54 % | 22 |
| 8. | 40 VP/50 iso-BMA/10 AS | 59 % | 67 % | 22 |

VP N-Vinylpyrrolidon, BA Butylacrylat, BMA Butylmethacrylat, AS Acrylsäure

**Tabelle 2**

| Bsp. Nr. | Zusammensetzung (Gew.-%) | Treibgasverträglichkeit (Propan/Butan 40:60, in Ethanol abs, 0°C) | Curl Retention (25°C, 90 % rel. Feuchte, 5 h) | K-Wert (2 gew.-%ig in Ethanol, 25°C) |
|---|---|---|---|---|
| 9. | 40 VP/50 tert.-BA/10 MAS | 68 % | 83 % | 30 |
| 10. | 38 VP/60 tert.-BA/2 MAS | 79 % | 83 % | 25 |
| 11. | 25 VP/70 tert.-BA/5 MAS | 77 % | 86 % | 24 |
| 12. | 20 VP/70 tert.-BA/10 MAS | 70 % | 88 % | 27 |

VP N-Vinylpyrrolidon, BA Butylacrylat, BMA Butylmethacrylat, MAS Methacrylsäure

Die Ergebnisse zeigen für die erfindungsgemäßen Terpolymeren in der Kombination Treibgasverträglichkeit und Haarfestigerwirkung überlegene Eigenschaften.

Treibgasverträglichkeiten unter 60 % und Curl Retention unter 50 % sind für die Praxis in der Regel nicht ausreichend. Das Vergleichsbeispiel 8 empfiehlt sich daher trotz einer guten Curl Retention nicht. Das Vergleichsbeispiel 7 bewegt sich an der unteren Grenze der Verwertbarkeit. Daß die erfindungsgemäßen Terpolymere mit tert.-Butylacrylat und t ert.-Butyl-methacrylat in der Kombination der untersuchten Eigenschaften die besten Werte ergeben, war nicht zu erwarten. Noch bessere Ergebnisse bringen die Zusammensetzungen mit Methacrylsäure für Acrylsäure.

Beispiele für Zubereitungen:

Beispiel 13

Aerosol-Haarspray
Polymeres gemäß Beispiel 11    2,0 %
2-Amino-2-methyl-propanol          0,04 %
Propan/Butan (15:85)          65,0 %
Ethanol, wasserfrei          32,96 %
Parfümöl          q.s.

Beispiel 14

Haarfestiger, rein wäßrig
Polymeres gemäß Beispiel 9          4,0 %
2-Amino-2-methyl-propanol          0,18 %
Wasser dest.          95,82 %
Parfümöl          q.s.

Beispiel 15

Haarfestiger, wäßrig-alkoholisch
Polymeres gemäß Beispiel 9          4,0 %
2-Amino-2-methyl-propanol          0,18 %
Ethanol          33,82 %
Wasser          62,0 %

**Patentansprüche**

1. Terpolymerisat, erhalten durch radikalische Polymerisation von

    20 bis 50 Gew.-%    Vinylpyrrolidon,
    40 bis 70 Gew.-%    tert.-Butylacrylat oder tert.-Butyl-methacrylat und
    2 bis 15 Gew.-%    Acrylsäure oder Methacrylsäure
    und dessen Carboxylgruppen gegebenenfalls zu 5 bis 100 % durch ein organisches Amin neutralisiert sind und das einen K-Wert von 10 bis 60 aufweist.

2. Terpolymerisat nach Anspruch 1, erhalten durch radikalische Polymerisation von

    20 bis 40 Gew.-%    Vinylpyrrolidon,
    40 bis 70 Gew.-%    tert.-Butylacrylat oder tert.-Butylmethacrylat und
    5 bis 10 Gew.-%,    Acrylsäure oder Methacrylsäure,
    dessen Carboxylgruppen zu 50 bis 90 % durch ein organisches Amin neutralisiert sind.

3. Haarbehandlungsmittel, enthaltend als Filmbildner ein Terpolymerisat gemäß Anspruch 1 oder 2 in einer Menge von 0,5 bis 15 Gew.-%, bezogen auf das Gesamtgewicht.

4. Haarbehandlungsmittel auf wäßriger Basis, enthaltend als Filmbildner ein Terpolymerisat gemäß Anspruch 1 oder 2 aus Vinylpyrrolidon, tert.-Butylacrylat und Methacrylsäure in einer Menge von 2 bis 10 Gew.-%, bezogen auf das Gesamtgewicht.

5. Haarsprayzubereitung, enthalten 0,5 bis 15 Gew.-% eines Terpolymerisats nach Anspruch 1, 10 bis 95 Gew.-% eines Lösungsmittels aus der Gruppe Aceton, Ethanol, Propanol, Isopropanol und 1-Methoxypropanol oder deren Gemische und

5 bis 90 Gew.-% eines Treibmittels, ausgewählt aus der Gruppe Propan, n-Butan, Isobutan, 2,2-Dimethylpropan, Isopentan und Dimethylether und deren Gemische.

6. Verwendung eines Terpolymerisats nach Anspruch 1 oder 2 als Filmbildner in Haarbehandlungsmitteln.

**Claims**

1. A terpolymer which is obtained by free radical polymerization of

from 20 to 50% by weight of vinylpyrrolidone,

from 40 to 70% by weight of tert-butyl acrylate or tert-butyl methacrylate and

from 2 to 15% by weight of acrylic acid or methacrylic acid and, if desired, from 5 to 100% of whose carboxyl groups have been neutralized by an organic amine and which has a K value of from 10 to 60.

2. A terpolymer as claimed in claim 1, which is obtained by free radical polymerization of

from 20 to 40% by weight of vinylpyrrolidone,

from 40 to 70% by weight of tert-butyl acrylate or tert-butyl methacrylate and

from 5 to 10% by weight of acrylic acid or methacrylic acid

and from 50 to 90% of whose carboxyl groups have been neutralized with an organic amine.

3. A hair treatment agent containing, as a film former, a terpolymer as claimed in claim 1 or 2 in an amount of from 0.5 to 15% by weight, based on the total weight.

4. An aqueous hair treatment agent containing, as a film former, a terpolymer as claimed in claim 1 or 2, of vinylpyrrolidone, tert-butyl acrylate and methacrylic acid, in an amount of from 2 to 10% by weight, based on the total weight.

5. A hair spray formulation containing from 0.5 to 15% by weight of a terpolymer as claimed in claim 1, from 10 to 95% by weight of a solvent from the group consisting of acetone, ethanol, propanol, isopropanol, 1-methoxypropanol and mixtures of these, and from 5 to 90% by weight of a propellant selected from the group consisting of propane, n-butane, isobutane, 2,2-dimethyl-propane, isopentane, dimethyl ether and mixtures of these.

6. Use of a terpolymer as claimed in claim 1 or 2 as a film former in hair treatment agents.

**Revendications**

1. Terpolymère, obtenu par polymérisation radicalaire de

20 à 50% en poids de vinylpyrrolidone,

40 à 70% en poids d'acrylate de tert. -butyle ou de méthacrylate de tert.-butyle et

2 à 15% en poids d'acide acrylique ou d'acide méthacrylique,

dont les groupements carboxyle sont éventuellement neutralisés, à raison de 5 à 100%, par une amine organique et qui présente une valeur K de 10 à 60.

2. Terpolymère selon la revendication 1, obtenu par polymérisation radicalaire de

20 a 40% en poids de vinylpyrrolidone,

40 à 70% en poids d'acrylate de tert.-butyle ou de méthacrylate de tert.-butyle et

5 à 10% en poids d'acide acrylique au d'acide méthacrylique,

dont les groupements carboxyle sont neutralisés, a raison de 50 à 90%, par une amine organique.

3. Produit de traitement des cheveux contenant, comme filmogène, un terpolymère selon la revendication 1 ou 2, dans une proportion de 0,5 a 15% en poids, par rapport au poids total.

4. Produit de traitement des cheveux à base aqueuse contenant, comme filmogène, un terpolymère selon la revendication 1 ou 2, composé de vinylpyrrolidone, d'acrylate de tert. butyle et d'acide méthacrylique, dans une proportion de 2 à 10% en poids par rapport à son poids total.

5. Préparation à pulvériser sur les cheveux, contenant de 0,5 à 15% en poids d'un terpolymère selon la

9

revendication 1, de 10 a 95% en poids d'un solvant du groupe comprenant l'acétone, l'éthanol, le propanol, l'isopropanol, le 1-méthoxypropanol ou des mélanges de ceux-ci, et de 5 à 90% en poids d'un agent propulseur choisi dans le groupe comprenant le propane, le n-butane, l'isobutane, le 2,2-diméthylpropane, l'isopentane, l'éther diméthylique et des mélanges de ceux-ci.

6. Utilisation d'un terpolymère selon la revendication 1 ou 2 comme filmogène dans des produits de traitement des cheveux.